# EUROPEAN PATENT SPECIFICATION

(11) **EP 1 539 074 B1**
(45) Date of publication and mention of the grant of the patent: **08.03.2006**
(21) Application number: 03736413.0
(22) Date of filing: 27.06.2003
(51) Int. Cl.: A61F 13/56, A61F 13/66

(54) **METHOD FOR CUTTING OUT DISCRETE FASTENING ELEMENTS AND APPLYING THESE TO AN ABSORBENT ARTICLE**
VERFAHREN ZUM AUSSCHNEIDEN DISKRETER BEFESTIGUNGSELEMENTE UND ZUM ANLEGEN DIESER ELEMENTE AN EINEN SAUGFÄHIGEN ARTIKEL
PROCEDE PERMETTANT DE DECOUPER DES ELEMENTS DE FIXATION DISCRETS ET D'APPLIQUER CEUX-CI SUR UN ARTICLE ABSORBANT

(30) Priority: 03.07.2002 SE 0202080
(43) Date of publication of application: 15.06.2005
(73) Proprietor: SCA Hygiene Products AB, 405 03 Göteborg (SE)
(72) Inventor: CORNELIUSSON, Helena, S-445 34 Bohus (SE)
(74) Representative: Hyltner, Jan-Olof
(86) International application number: PCT/SE2003/001133
(87) International publication number: WO 2004/004620

(56) References cited:
- WO-A1-02/00152
- WO-A1-98/43586
- US-B1- 6 234 229

## Description

### TECHNICAL FIELD

The present invention relates to a method for cutting discrete fastening elements out of a web of fastening element material and attaching the cut-out fastening elements to a web of surface layer material forming part of a process line for manufacturing absorbent articles, such as diapers, pant diapers or incontinence pads, and also to an absorbent article provided with such fastening elements.

### BACKGROUND OF THE INVENTION

It has to be possible for diapers, pant diapers, incontinence pads and similar disposable articles to fit different sizes and shapes of wearer in order for a reasonable number of sizes of article to be sufficient for the requirements of the wearers. Traditionally, it has been possible for the size of the waist of diapers to be adjusted by virtue of the possibility of the side portions of the diaper overlapping one another to a varying degree. To this end, the diaper comprises fastening tapes which can be fastened in different places along the circumference of the waist, either directly to the surface layer material of the diaper or to a strip made of a material which allows the fastening tape to be fastened a number of times. Another possibility used for adapting an article according to the above to several sizes and for improving the fit of the article is to make the waistband either completely or partly elastic. Many modern absorbent articles according to the above make use of both these possibilities and therefore have both adjustable fastening elements and waist elastic. Furthermore, openable and reclosable pant diapers are known, in which the fastening, elements are designed in such a manner that the side portions are fastened to one another along their entire length. The fastening elements consist of male and female elements of hook and loop means or tape/plastic strip. The female elements constitute receiving elements, and their extent in the circumferential direction determines the adjustment length.

The materials used for fastening elements are often considerably more expensive than other materials included in an absorbent article, and the cost of the fastening elements therefore constitutes a not inconsiderable proportion of the overall cost of the article. Moreover, the presence of the fastening elements also influences the characteristics of the product, such as rigidity, breathability, flexibility. It is therefore an advantage to use as little fastening element material as possible in the manufacture of absorbent articles.

It is an object of the invention to provide a method in which fastening elements can be cut out and applied to an absorbent article without waste of fastening element material. It is also an object of the invention to produce fastening elements which allow a certain adjustment length of the waistband with a lower consumption of fastening element material than in previously known absorbent articles.

### SUMMARY OF THE INVENTION

These objects are achieved by means of a method for cutting discrete fastening elements out of a web of fastening element material and attaching the cut-out fastening elements to a web of surface layer material forming part of a process line for manufacturing absorbent articles, such as diapers, pant diapers or incontinence pads, in which the individual articles in the process line have their front edges facing one another, characterized by the following steps: sequences of four fastening elements are cut out of a web of fastening element material, which elements each have a different length on their mutually opposite short sides and long sides, and of which elements the first fastening element and fourth fastening element in such a sequence have longest short sides which extend in prolongation of one another and of the short short sides of the second fastening element and third fastening element, and the second fastening element and third fastening element have longest short sides which extend in prolongation of one another and of the short short sides of the first fastening element and fourth fastening element, in addition to which the short long side of each fastening element is at right angles to the short sides, after which the first and fourth fastening elements on the one hand and the second and third fastening elements on the other hand are attached to a web of surface layer material with the short sides extending along the same transverse line of the web of surface layer material, with the shortest long sides located next to the side edges of the web of surface layer material and extending in its longitudinal direction and with the longest short sides of the first and fourth fastening elements facing the longest short sides of the second and third fastening elements so that the first and fourth fastening elements on the one hand and the second and third fastening elements on the other hand are attached to successive absorbent articles in the process line. In such an embodiment, the waist size can be reduced to an extent which is determined by the length of the long short sides of the fastening elements by virtue of a second fastening element which interacts with the fastening elements being fastened obliquely on the fastening elements. At the same time, the size of the leg openings can be reduced to an extent which is determined by the length of the short short sides. Furthermore, no waste of fastening element material occurs in manufacture, and, after cutting-out, the fastening elements have their long short sides facing in the correct direction and can be applied to the articles without having to be rotated.

In a preferred embodiment, the material in the web of surface layer material consists of a liquidtight plastic material. Alternatively, the material in the web of surface layer material can consist of a liquid-permeable material.

The fastening elements are preferably attached to the surface layer material by means of thermal or ultrasonic welding, but gluing can also be used.

The fastening elements preferably comprise loop means designed to interact with hook means of a second fastening element which interacts with the fastening elements, but the fastening elements can also be made of plastic material in order to interact with second fastening elements of adhesive type.

The invention also relates to an absorbent article, such as a diaper, a pant diaper or an incontinence pad, comprising an absorption body enclosed between an inner liquid-permeable surface layer and an outer liquidtight surface layer and also a front portion, a rear portion and a crotch portion which lies between these portions and includes and is delimited by the leg openings of the article, and also fastening means for openably and reclosably fastening mutually opposite side parts of the front portion and the rear portion to one another, which fastening means consist of interacting pairs of fastening elements, which extend along the side edges of the front and rear portions, characterized in that one of the elements in the pairs of fastening elements tapers continuously from the end of the front portion or rear portion towards the leg opening.

### LIST OF FIGURES

The invention will now be described with reference to accompanying figures, in which:
Fig. 1 shows diagrammatically a perspective view of a diaper according to an embodiment of the invention,
Fig. 2 illustrates diagrammatically the various steps in the manufacture of the diaper in Figure 1,
Fig. 3 shows a portion of Figure 2 on larger scale,
Fig. 4 shows an alternative design of the fastening element material web and the cutting-out of individual elements therefrom,
Fig. 5 shows diagrammatically a fastening means web which can be used in the manufacture of diaper pants according to an embodiment of the invention,
Fig. 6 shows diagrammatically the final steps in the manufacture of a pant diaper,
Figs 7a and b show diagrammatically a fastening means according to an embodiment of the invention with the male element placed in different positions, and

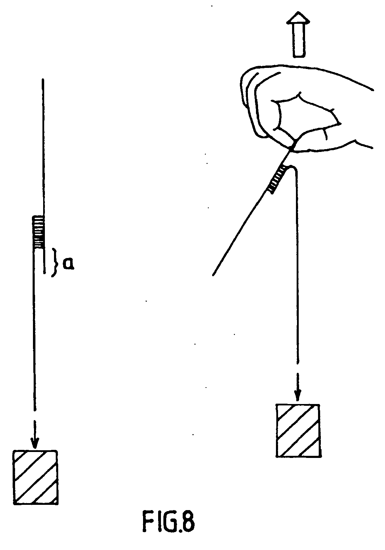

Fig. 8 shows a method of testing the childproofness of a connection.

### DESCRIPTION OF EMBODIMENTS

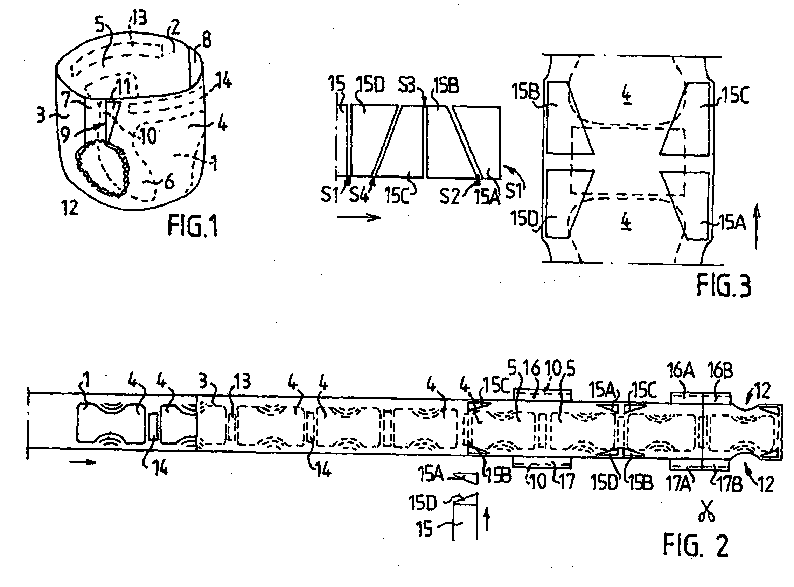

Figure 1 shows a diaper according to a preferred embodiment of the invention. The pant diaper shown in Figure 1 comprises an absorption body 1 enclosed between an inner surface layer 2 made of liquid-permeable material and an outer surface layer 3 made of liquidtight material. The surface layers 2 and 3 are interconnected by gluing or welding in parts lying outside the absorption body. The unit formed by the surface layers and the absorption body has a front portion 4, a rear portion 5 and an intermediate narrower crotch portion 6 which is delimited by the leg openings of the diaper. The diaper also comprises side panels 7, 8 which are attached to the side edges of the rear portion.

The side panels 7, 8 bear one fastening element of unfastenable and reclosable fastening means 9 which connect the side edges of the panels 7, 8 to the front portion 4, and the second fastening element is attached to the front portion 4 of the diaper. In the example shown, the fastening means 9 is of the hook and loop type, that is to say one fastening element 10, the male element, is provided with a large number of hooks which project from the surface of the fastening element, and the other fastening element 11, the female element, is provided with a large number of loops into which the hooks fit. The fastening means 9 extend along at least 70% of the side edges of the front portion 4.

The side panels 7, 8 are elastic and are preferably made from elastic material apart from in the parts which comprise the fastening elements 10, which parts can consist of inelastic material. The elastic material can consist of elastomers made from block copolymers, such as polyurethanes, copolyether esters, polyamidepolyether block copolymers, ethylene-vinylacetates (EVAs) and the like, including polyurethanes available from E.I Du Pont de Nemours Co., USA under the name LYCRA® (also known as spandex); elastomeric styrenebutadiene copolymers, including those such as KRATON® material, which are available from Shell Chemical Company of Houston, Texas, USA; tetrablock copolymers, including elastomeric styrene-poly(ethylene-propylene) block copolymers available from Shell Chemical Company of Houston, Texas, USA under the trade name KRATON®; polyamides including polyether block amides available from Ato Chemical Company, USA under the trade name PEBAX®; polyesters, such as those available from E.I Du Pont de Nemours Co. under the trade name HYTREL®; single-site or metallocene-catalyzed polymers, including single-site or metallocene-catalyzed polyolefins with a density of less than around 0.89 g/cm³ from Dow Chemical Co., USA under the trade name AFFINITY®; and natural and synthetic rubber.

The purpose of the elastic side panels is to give the pant diaper a good fit. The side panels are dimensioned so as to provide the necessary elastic force but no more. It is therefore conceivable for the side panels also to have parts made of inelastic material in places other than at the fastening elements. Such an inelastic material can consist of a non-woven material, for example a spunbond non-woven, a carded non-woven, a meltblown non-woven or a non-woven laminate, for example a spunbond-meltblown-spunbond (SMS) laminate. The fibres used for constructing the non-woven materials can consist of fibres made of polyolefins, for example polyethylene or polypropylene, or of polyester. The non-woven material can also consist of a mixture of a number of different types of fibre or of fibres which consist of a number of different polymers, what are known as copolymers. It is also possible for the inelastic material to consist of a plastic film.

It is also conceivable to make use of, for example, two non-woven layers, between which elastic materials, elastic bands or elastic threads in a stretched state are attached, as the material for the side panels. In such a material, the elastic and inelastic parts of the side panels consist of portions with and without such elastic materials.

As can be seen from Figure 1, the diaper also comprises leg elastic 12 which consists of the lower portion of the side panels 7, 8 and one or more elastic threads which are arranged between the surface layers 2 and 3 and are attached to these in a stretched state on both sides of the absorption body 1. Elastic bands 13, 14, which are attached to both surface layers 2, 3 in a stretched state, also form waist elastic in the front and rear portions of the diaper.

The liquid-permeable surface layer 2 consists of, for example, a non-woven made of spunbond polypropylene. Other materials which are used for liquid-permeable surface layers, what are known as top sheets, for absorbent articles, such as non-wovens made of synthetic and/or natural fibres, perforated plastic films or laminates of such materials, can of course also be used as the surface layer 2.

The liquidtight outer surface layer 3 can consist of a plastic layer, preferably of a breathable type, or a laminate consisting of a plastic layer and a non-woven. All materials which are used as what are known as backing layers for absorbent articles can be used.

The absorption body 1 preferably comprises a layer of cellulose fibres with or without superabsorbents and/or binding fibres mixed in. Other materials, such as foamed materials or moss, can also be used. The absorption body can also be constructed from a number of layers and advantageously comprises a layer of a material with high permeability, for example a wadding layer.

The fastening elements 11 are parallel-trapeziform with parallel short sides 11A, 11B, a short long side 11C, which extends at right angles to the short sides, and a long long side 11D, which is inclined relative to the short long side 11C. The fastening elements 11 are attached to the outside of the surface layer 2 in the side edge portions of the front portion 4 with the long long sides facing one another and with the long short sides positioned next to the waist edge of the diaper. The fastening element 10 which interacts with the fastening elements 11 preferably has a rectangular shape and a width which is the same as or smaller than the width of the shortest short side of the fastening elements 11. In the longitudinal direction of the diaper, the fastening elements 10, 11 extend along the entire side edge of the front portion.

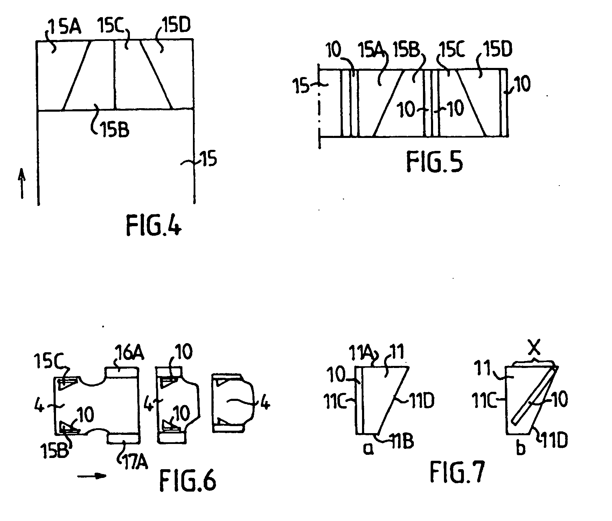

Figures 7a and b show the male element 10 in its two extreme positions on the female element 11. In the position shown in Figure 7a, in which the element 10 extends along the short long side 11C of the element 11, the waist size of the diaper is at its maximum whereas, in the position shown in Figure 7b, it is at its minimum. In Figure 7b, the fastening element 10 has been rotated so that its edge lying next to the waistband is located at as great a distance as possible from the short long side 11C of the element 11. In this way, the waist size is reduced by the length x in Figure 7b, that is to say, if the fastening elements 10 of both fastening means 9 are positioned in a manner corresponding to Figure 7b, the waist size is reduced by 2x.

By virtue of the fact that the short short side 11B of the fastening element 11 is longer than the width of the fastening element 10, a small adjustment possibility for reducing the size of the leg opening 12 is also obtained.

By utilizing parallel-trapeziform fastening elements of the female type, the quantity of fastening element material for bringing about a certain adjustment length of the waist size can be substantially reduced in a simple manner, which means a considerable cost saving. Furthermore, such material is present in only the places where it is required, which means that its influence on the product in terms of, for example, increased local rigidity and reduced local flexibility is reduced.

In the embodiment described, the fastening means consist of interacting mechanical fastening elements, but it is also conceivable to use adhesive male elements which interact with female elements made of material which allows unfastening of the adhesive without the material being damaged or the adhesive force being reduced appreciably.

An embodiment of a method for manufacturing diapers according to Figure 1 will now be described with reference to Figures 2 and 3. In order to simplify comparison with the diaper in Figure 1, components in Figures 2 and 3 have been given the same reference numbers as corresponding components of the finished diaper in Figure 1. For example, the web of liquid-permeable material in Figures 2 and 3 has been given the same reference number as the surface layer 2 of the finished diaper.

A diaper according to Figure 1 is manufactured in the manner described below.

Absorption bodies 1 are applied to a moving material web 2 of liquid-permeable material by means of, for example, a transfer wheel, onto which absorption bodies 1 formed in a mat-forming wheel have been distributed. The feed direction of the material web 2 is indicated by an arrow in Figure 2. If the forming of absorption bodies 1 can take place synchronously with the advance of the material web 2, the transfer wheel can be omitted, and the wheel can consist of a mat-forming wheel. The moulds of the mat-forming wheel are suitably designed so that the absorption bodies which leave the mat-forming wheel have front ends and rear ends facing one another. If this is not the case, the transfer from the mat-forming wheel is effected in such a manner that every other absorption body is rotated by 180° before the absorption bodies are distributed onto the transfer wheel. The absorption bodies 1 are therefore distributed onto the material web 2 in a row with front portions and rear portions of adjacent absorption bodies facing one another. The material web 2 and components added to it subsequently therefore constitute a web of consecutive diaper blanks in different manufacturing stages.

Elastic threads are then applied along the edges of the future leg openings 12, and elastic bands 13, 14 are applied in the interspaces between the absorption bodies 1 in the row of laid-out absorption bodies.

A material web 3 of liquidtight material is then applied on top of the row of absorption bodies 1. Directly before application, the material web 3 passes through a gluing unit and is attached, by means of a roller pair, to the material web 2 in parts lying outside the absorption bodies 1. The material web 3 may also be attached to the rear side of every absorption body 1. It may be pointed out that the manufacturing steps described so far are conventional and well-known to the person skilled in the art.

Parallel-trapeziform fastening elements 15A-15D are then applied to the liquidtight surface layer 3 in the front portions 4 in a manner which will be described in greater detail below with reference to Figure 3. The material pieces 16, 17 are then attached to the side edges of the rear portions 5, after which individual diapers with side panels 16A, 17A and, respectively, 16B, 17B and leg openings are cut out of the web of consecutive diaper blanks. In the figure, this cutting-out is indicated by a scissors symbol. After having been formed into finished diapers, the diapers are conveyed to a packing station.

Strips of fastening element material of the male type, that is to say comprising hook means, are attached to the side panel blanks 16, 17 either before or after these are attached to the web of consecutive diaper blanks. The fastening element strips are preferably applied to the side panel blanks 16, 17 in advance before these are attached to the web of consecutive diaper blanks.

Figure 3 shows on larger scale than in Figure 2 how a sequence of fastening elements 15A-15D is cut out of a web 15 of fastening element material and applied to a web of diaper blanks. The feed directions of the two webs are indicated by arrows in Figure 3. The embodiment shown in Figure 3 differs from that shown in Figure 2 only in that the web 15 runs to the left of the diaper blank web in Figure 3 and the leg openings in the diaper blank web in Figure 3 are already cut out. However, these differences have no significance for the method of cutting-out and applying fastening elements illustrated in Figures 2 and 3.

In order to produce the elements 15A-15D, the web 15 is cut alternately with cuts S1, S3, which extend entirely in the transverse direction of the web at right angles to the feed direction, and with cuts S2, S4, which extend in an inclined manner in relation to the transverse direction and the feed direction. The inclined cuts S2, S4 are inclined in different directions but at the same angle to the transverse direction. In this way, sequences of four parallel-trapeziform fastening elements 15A-15D are produced, in which the first element 15A and fourth element 15D on the one hand and the second element 15B and third element 15C on the other hand have their long short sides along the same side edge of the web 15. It may be pointed out that, in this text, short sides mean those sides of the parallel trapezia which are parallel to one another even if these sides should be longer than one or both of the long sides.

The cut-out fastening elements are then transferred in a suitable manner to the diaper blank web and are positioned in the intended places in the two opposite front portions 4 of the diaper blanks in the diaper blank web. The transfer can take place by means of four arms, each having gripping means, for example suction means, for taking hold of a fastening element, which move the elements 15A-15D to the intended places. This movement preferably takes place synchronously with the feed of the diaper blank web so that the elements 15A-15D only have to be moved in the lateral direction and the vertical direction. In such an embodiment, which is illustrated diagrammatically in Figure 2, first the second and third elements 15B and 15C are moved laterally and then pressed firmly against the diaper blank web, after which the first and fourth elements 15A and 15D are moved laterally. The attachment of the elements 15A-15D can take place by means of thermal or ultrasonic welding or by means of gluing. In the event of gluing, the elements 15A-15D suitably pass through a gluing unit during their movement to the diaper blank web. It is of course also possible to carry out the movement of the elements 15A-15D simultaneously for all the elements or separately, that is to say each element is moved directly after cutting-out, and with a movement component in the feed direction of the diaper web.

For reasons of clarity, the cutting-out of the elements 15A-15D is shown as taking place in the lateral direction outside the diaper blank web but, if space allows, it is also possible for cutting-out to take place above the diaper blank web in order to reduce the length of the necessary lateral movements of the fastening elements.

In the embodiment shown in Figure 3, the cutting-out of fastening elements takes place from a material web which has a width which is the same as the height of the fastening elements, that is to say the length of the long side which is to extend along the side edges of the front portion of the diaper. Figure 4 shows an embodiment where the cutting-out of the elements 15A-15D takes place from a material web which has a width which is the same as the length of a sequence of cut-out elements 15A-15D. In such a web, transverse cuts are made at a distance from one another which corresponds to the height of the fastening elements, after which one entirely longitudinally directed cut and two longitudinal cuts inclined towards one another are made. It is of course possible to make the longitudinal cuts before the transverse cut if so desired. Otherwise, neither the cutting-out nor the application differ from what was described with reference to Figures 2 and 3.

Figure 5 shows diagrammatically a fastening element web 15 which has been provided with a series of successively arranged strips of male element material, each strip having double the width of a male element 10, located at a distance from one another which corresponds to the combined length of the short sides of a trapeziform element 15A-15D. Furthermore, the tops of some of the hook means of the male elements are firmly connected to the female element web, for example by thermal or ultrasonic welding or by gluing. It can be seen that fastening elements of the female type provided with male elements 10 in accordance with what is shown in Figure 5 can also be manufactured in a fastening element web according to Figure 4 simply by arranging male element strips along the longitudinal edges of such a web and along its centre line.

A design according to Figure 5 is suitable if a pant diaper with childproof unfastenable and reclosable fastening means for interconnection of the side edges of the front and rear portions is to be manufactured. A childproof connection that is difficult or impossible for an infant to open but easy for an adult to open is obtained. It has of course been found that infants like to tamper with their pant diapers and that there is therefore a need for a childproof connection. The number of firmly connected points should be selected so that the force which is required to open such a connection is greater than 4 N, preferably greater than 6 N, more preferably greater than 8 N and most preferably greater than 10 N but smaller than 20 and preferably smaller than 15 N in order to ensure childproofness and make the connection easy to open for an adult. The opening force can be measured simply in the manner shown diagrammatically in Figure 7 by connecting a weight to that part of the two parts of the connection which is overlapped, and then taking hold of the grip part of the overlapping part and lifting the connection. If the weight remains hanging from the connection for more than 30 seconds without the connection coming undone, the connection is considered to hold for the weight concerned.

The blanks for pant diapers according to the invention can suitably be manufactured in the same way as described with reference to Figures 2-5. Figure 6 illustrates the final steps of such manufacture and Figure 6 can be considered to constitute a supplement to Figure 2, that is to say Figure 6 begins where Figure 2 ends, namely with the cutting-out of diapers. The pant diaper blank shown in Figure 6 corresponds to a diaper produced according to Figure 2 with the modification that it is provided with fastening elements in accordance with Figure 5, that is to say the fastening elements 10 are already fastened to the female elements 15 when these are cut out. In order to produce a pant diaper from the blank shown in Figure 6, the pant diaper blank is folded around a transverse axis so that the front and rear portions come to lie edge to edge. The side panels 16, 17 are then folded in over the side edges of the front portion 4, and the folded-in parts of the side panels 16, 17 are finally attached in a suitable manner to the fastening elements 10, for example by gluing or thermal or ultrasonic welding.

In the embodiment described, the web 3 of liquidtight material is laid on top of the web 2 of liquid-permeable material after absorption bodies have been distributed onto the latter. It is of course possible for the webs to be the other way round, so that absorption bodies are distributed onto the web of liquidtight material and the web of liquid-permeable material is applied last. In such an application, the fastening elements can be attached to the web 3 before absorption bodies are applied to the latter.

It is of course also conceivable for the fastening elements to be the other way round in such a manner that the rectangular and elongate male elements are attached to the outside of the front portion and the parallel-trapeziform female elements are attached to the inside of the rear portion, in which case adjustment is brought about by the female elements being placed in different positions on the male elements. However, this is not preferred. It is also conceivable to place the female elements on the outside of the rear portion and the male elements on the inside of the front portion.

The method described can of course be modified within the scope of the invention. For example, the side panels 16, 17 can be applied to the material web 2 before the material web 3 is applied, so that the side panels are arranged between the material webs 2 and 3 and are attached to both these webs. The side panels can also be attached to the liquid-permeable surface layer. The absorption bodies 1 can be supplemented by further layers by such layers being applied on top of the bodies 1 by additional transfer or mat-forming wheels being added to the process line shown in Figure 2. Means for providing liquid barriers, what are known as standing gathers, can also be added to the process line. The leg elastic and the waist elastic, which preferably extend along the front and rear edges of the pant diaper, do not have to be arranged between the material webs 2 and 3 but can be applied to one of these webs. The absorption bodies can have a shape different to that shown, for example rectangular or hourglass-shaped. Types of mechanical fastening element other than hook and loop means can be used, for example various types of snap connections, which can also be childproofed. The components included in the process line described are of such a type as is normally used in manufacturing diapers, pant diapers and similar articles and can be replaced by other components with the same function. The invention is therefore to be limited only by the content of the accompanying patent claims.

## Claims

1. Method for cutting discrete fastening elements (15A-15D) out of a web (15) of fastening element material and attaching the cut-out fastening elements to a web (3) of surface layer material forming part of a process line for manufacturing absorbent articles, such as diapers, pant diapers or incontinence pads, in which the individual articles in the process line have their front edges facing one another, **characterized by** the following steps: sequences of four fastening elements (15A-15D) are cut out of a web (15) of fastening element material, which elements each have a different length on their mutually opposite short sides and long sides, and of which elements the first fastening element (15A) and fourth fastening element (15D) in such a sequence have longest short sides which extend in prolongation of one another and of the short short sides of the second fastening element (15B) and third fastening element (15C), and the second fastening element and third fastening element have longest short sides which extend in prolongation of one another and of the short short sides of the first fastening element and fourth fastening element, in addition to which the short long side of each fastening element is at right angles to the short sides, after which the first and fourth fastening elements on the one hand and the second and third fastening elements on the other hand are attached to a web (3) of surface layer material with the short sides extending along the same transverse line of the web of surface layer material, with the shortest long sides located next to the side edges of the web of surface layer material and extending in its longitudinal direction and with the longest short sides of the first and fourth fastening elements facing the longest short sides of the second and third fastening elements so that the first and fourth fastening elements on the one hand and the second and third fastening elements on the other hand are attached to successive absorbent articles in the process line.

2. Method according to Claim 1, **characterized in that** the material in the web (3) of surface layer material consists of a liquidtight plastic material.

3. Method according to Claim 1, **characterized in that** the material in the web of surface layer material consists of a liquid-permeable material.

4. Method according to any one of Claims 1-3, **characterized in that** the fastening elements (15A-15D) are attached to the surface layer material by means of thermal or ultrasonic welding.

5. Method according to any one of Claims 1-3, **characterized in that** the fastening elements (15A-15D) are attached to the surface layer material by means of gluing.

6. Method according to any one of Claims 1-5, **characterized in that** the fastening elements (15A-15D) comprise loop means.

7. Method according to any one of Claims 1-5, **characterized in that** the fastening elements are made of plastic material.

8. Absorbent article, such as a diaper, a pant diaper or an incontinence pad, comprising an absorption body (1) enclosed between an inner liquid-permeable surface layer (2) and an outer liquidtight surface layer (3) and also a front portion (4), a rear portion (5) and a crotch portion (6) which lies between these portions and includes and is delimited by the leg openings (12) of the article, and also fastening means (9) for openably and reclosably fastening mutually opposite side parts of the front portion (4) and the rear portion (5) to one another, which fastening means (9) consist of interacting pairs of fastening elements (10, 11), which extend along the side edges of the front and rear portions, **characterized in that** one of the elements (11) in the pairs of fastening elements tapers continuously from the end of the front portion (4) or rear portion (5) towards the leg opening (12).

9. Article according to Claim 8, **characterized in that** the tapering fastening elements (11) are parallel-trapeziform with two parallel short sides (11A, 11B), a short long side (11C), which extends at right angles to the short sides, and a long long side (11D), which is located at a greater distance from the closest side edge of that portion (4) of the article to which the element is attached than the short long side.

10. Article according to Claim 9, **characterized in that** the fastening element (10) which interacts with the tapering fastening element (11) is elongate and rectangular, **in that** the width of the shortest short side (11B) of the tapering fastening element (11) is the same as or greater than the width of the elongate fastening element, and **in that** the length of the elongate fastening element (10) and the length of the shortest long side (11C) of the tapering fastening element lie between 70-100% of the length of the side edges of the front portion (4).

## Revendications

1. Une méthode pour découper des éléments d'attache discrets (15A-15D) dans une bande continue (15) de matière d'éléments d'attache et pour fixer les éléments d'attache découpés sur une bande continue (3) de matière de couche superficielle faisant partie d'une chaîne de fabrication pour la production d'articles absorbants, tels que des couches, des couches-culottes ou des garnitures d'incontinence, dans laquelle les articles individuels dans la chaîne de production ont leurs bords avant orientés les uns vers les autres, **caractérisée par** les étapes suivantes: des séquences de quatre éléments d'attache (15A-15D) sont découpées dans une bande continue (15) de matière d'éléments d'attache, lesquels éléments ont chacun une longueur différente sur leurs côtés courts et côtés longs mutuellement opposés, et de ces éléments le premier élément d'attache (15A) et le quatrième élément d'attache (15D) dans une telle séquence ont des côtés courts plus longs qui s'étendent dans le prolongement l'un de l'autre ainsi que des courts côtés courts du deuxième élément d'attache (15B) et troisième élément d'attache (15C), et le deuxième élément d'attache et le troisième élément d'attache ont des côtés courts les plus longs qui s'étendent dans le prolongement l'un de l'autre ainsi que des courts côtés courts du premier élément d'attache et du quatrième élément d'attache, en plus de quoi le court côté long de chaque élément d'attache est en angle droit par rapport aux côtés courts, après quoi les premier et quatrième éléments d'attache d'une part et les deuxième et troisième éléments d'attache d'autre part sont fixés à une bande continue (3) de matière de couche superficielle avec les côtés courts s'étendant le long de la même ligne transversale de la bande continue de matière de couche superficielle, avec les côtés longs les plus courts situés auprès des bords latéraux de la bande continue de matière de couche superficielle et s'étendant dans sa direction longitudinale et avec les côtés courts les plus longs des premier et quatrième éléments d'attache orientés vers les côtés courts les plus longs des deuxième et troisième éléments d'attache afin que les premier et quatrième éléments d'attache d'une part et les deuxième et troisième éléments d'attache d'autre part soient fixés à des articles absorbants successifs dans la chaîne de production.

2. Une méthode selon la revendication 1, **caractérisée en ce que** la matière dans la bande continue (3) de matière de couche superficielle consiste d'une matière plastique étanche aux liquides.

3. Une méthode selon la revendication 1, **caractérisée en ce que** la matière dans la bande continue de matière de couche superficielle consiste d'une matière perméable aux liquides.

4. Une méthode selon l'une quelconque des revendications 1 à 3, **caractérisée en ce que** les éléments d'attache (15A-15D) sont fixés à la matière de couche superficielle au moyen de soudage thermique ou ultrasonique.

5. Une méthode selon l'une quelconque des revendications 1 à 3, **caractérisée en ce que** les éléments d'attache (15A-15D) sont fixés à la matière de couche superficielle au moyen de collage.

6. Une méthode selon l'une quelconque des revendications 1 à 5, **caractérisée en ce que** les éléments d'attache (15A-15D) comportent des moyens à boucle.

7. Une méthode selon l'une quelconque des revendications 1 à 5, **caractérisée en ce que** les éléments d'attache sont faits de matière plastique.

8. Un article absorbant, tel qu'une couche, une couche-culotte ou une garniture d'incontinence, comportant un corps absorbant (1) enfermé entre une couche superficielle intérieure perméable aux liquides (2) et une couche superficielle extérieure étanche aux liquides (3) ainsi qu'une portion avant (4), une portion arrière (5) et une portion d'entre-jambes (6) qui repose entre ces portions et comprend et est délimitée par les ouvertures de jambes (12) de l'article, ainsi que des moyens d'attache (9) pour fixer, tout en pouvant les ouvrir et les refermer, les parties latérales mutuellement opposées de la portion avant (4) et de la portion arrière (5) l'une à l'autre, lesquels moyens d'attache (9) consistent de paires d'éléments d'attache interdépendants (10, 11), qui s'étendent le long des bords latéraux des portions avant et arrière, **caractérisé en ce qu'**un des éléments (11) des paires d'éléments d'attache s'effile de façon continue à partir de l'extrémité de la portion avant (4) ou de la portion arrière (5) vers l'ouverture de jambe (12).

9. Un article selon la revendication 8, **caractérisé en ce que** les éléments d'attache effilés (11) sont en forme de trapèzes parallèles avec deux côtés courts parallèles (11A, 11B), un court côté long (11C), qui s'étend en angle droit par rapport aux côtés longs, et un long côté long (11D), qui est situé à une plus grande distance du bord latéral le plus rapproché de cette portion (4) de l'article auquel l'élément est fixé que le court côté long.

10. Un article selon la revendication 9, **caractérisé en ce que** l'élément d'attache (10) qui est interdépendant de l'élément d'attache effilé (11) est allongé et rectangulaire, **en ce que** la largeur du côté court le plus court (11B) de l'élément d'attache effilé (11) est la même ou plus grande que la largeur de l'élément d'attache allongé, et **en ce que** la longueur de l'élément d'attache allongé (10) et la longueur du côté long le plus court (11C) de l'élément d'attache effilé se situe entre 70-100% de la longueur des bords latéraux de la portion avant (4).

## Patentansprüche

1. Verfahren zum Ausschneiden separater Befestigungselemente (15A-15D) aus einer Bahn (15) aus Befestigungselementmaterial und Anbringen der ausgeschnittenen Befestigungselemente auf einer Bahn (3) aus Oberflächenlagenmaterial, die einen Teil einer Fertigungsstraße zum Herstellen absorbierender Gegenstände, wie beispielsweise Windeln, Höschenwindeln oder Inkontinenzpads, bildet, wobei in der Fertigungsstraße die Vorderkanten der einzelnen Gegenstände einander zugewandt sind, **gekennzeichnet durch** die folgenden Schritte: Sequenzen von vier Befestigungselementen (15A-15D) werden aus einer Bahn (15) aus Befestigungselementmaterial ausgeschnitten, wobei die Elemente jeweils eine unterschiedliche Länge an ihren entgegengesetzten kurzen Seiten und langen Seiten aufweisen und wobei von diesen Elementen das erste Befestigungselement (15A) und das vierte Befestigungselement (15D) einer derartigen Sequenz längste kurze Seiten aufweisen, die sich in der Verlängerung voneinander und der kurzen kurzen Seiten des zweiten Befestigungselements (15B) und dritten Befestigungselements (15C) erstrecken, und wobei das zweite Befestigungselement und das dritte Befestigungselement längste kurze Seiten aufweisen, die sich in der Verlängerung voneinander und der kurzen kurzen Seiten des ersten Befestigungselements und vierten Befestigungselements erstrecken, wobei zusätzlich dazu die kurze lange Seite jedes Befestigungselements im rechten Winkel zu den kurzen Seiten verläuft, wonach das erste und vierte Befestigungselement einerseits und das zweite und dritte Befestigungselement andererseits an einer Bahn (3) aus Oberflächenmaterial angebracht werden, wobei sich die kurzen Seiten entlang der gleichen Querlinie der Bahn aus Oberflächenlagenmaterial erstrecken, wobei die kürzesten langen Seiten nahe der Seitenkanten der Bahn aus Oberflächenlagenmaterial angeordnet sind und sich in ihrer Längsrichtung erstrecken, und wobei die längsten kurzen Seiten des ersten und vierten Befestigungselements den längsten kurzen Seiten des zweiten und dritten Befestigungselements zugewandt sind, so dass das erste und vierte Befestigungselement einerseits und das zweite und dritte Befestigungselement andererseits in der Fertigungsstraße an aufeinanderfolgenden absorbierenden Gegenständen angebracht werden.

2. Verfahren nach Anspruch 1, **dadurch gekennzeichnet, dass** das Material der Bahn (3) aus Oberflächenlagenmaterial aus einem flüssigkeitsdichten Kunststoffmaterial besteht.

3. Verfahren nach Anspruch 1, **dadurch gekennzeichnet, dass** das Material der Bahn aus Oberflächenlagenmaterial aus einem flüssigkeitsdurchlässigen Material besteht.

4. Verfahren nach einem der Ansprüche 1 bis 3, **dadurch gekennzeichnet, dass** die Befestigungselemente (15A-15D) mittels thermischem Schweißen oder Ultraschallschweißen an dem Oberflächenlagenmaterial angebracht sind.

5. Verfahren nach einem der Ansprüche 1 bis 3, **dadurch gekennzeichnet, dass** die Befestigungselemente (15A-15D) mittels Kleben an dem Oberflächenlagenmaterial angebracht sind.

6. Verfahren nach einem der Ansprüche 1 bis 5, **dadurch gekennzeichnet, dass** die Befestigungselemente (15A-15D) Schlaufenmittel umfassen.

7. Verfahren nach einem der Ansprüche 1 bis 5, **dadurch gekennzeichnet, dass** die Befestigungselemente aus einem Kunststoffmaterial gebildet sind.

8. Absorbierender Gegenstand, wie eine Windel, eine Höschenwindel oder ein Inkontinenzpad, umfassend einen Absorptionskörper (1), der zwischen einer inneren flüssigkeitsdurchlässigen Oberflächenlage (2) und einer äußeren flüssigkeitsdichten Oberflächenlage (3) eingeschlossen ist, und auch einen Vorderabschnitt (4), einen Hinterabschnitt (5) und einen Schrittabschnitt (6), der zwischen diesen Abschnitten liegt, Beinöffnungen (12) des Gegenstands umfasst und durch diese begrenzt ist, und ferner eine Befestigungseinrichtung (9) zum lösbaren und wiederverschließbaren Befestigen gegenüberliegender Seitenteile des Vorderabschnitts (4) und des Hinterabschnitts (5) aneinander, wobei die Befestigungseinrichtung (9) aus miteinander zusammenwirkenden Paaren von Befestigungselementen (10, 11) besteht, die sich entlang der Seitenkanten des Vorder- und Hinterabschnitts erstrecken, **dadurch gekennzeichnet, dass** eines dieser Elemente (11) des Paars Befestigungselemente von dem Ende des Vorderabschnitts (4) oder dem Hinterabschnitt (5) in Richtung der Beinöffnung (12) kontinuierlich verjüngt ist.

9. Gegenstand nach Anspruch 8, **dadurch gekennzeichnet, dass** die sich die verjüngenden Befestigungselemente (11) parallel trapezförmig zu zwei parallelen kurzen Seiten (11A, 11B), einer kurzen langen Seite (11C), die sich im rechten Winkel zu den kurzen Seiten erstreckt, und einer langen langen Seite (11D), die in einem größeren Abstand von der nächsten Seitenkante des Abschnitts (4) des Gegenstands angeordnet ist, an dem das Element angebracht ist, als von der kurzen langen Seite, ist.

10. Gegenstand nach Anspruch 9, **dadurch gekennzeichnet, dass** das Befestigungselement (10), das mit dem sich verjüngenden Befestigungselement (11) zusammenwirkt, länglich und rechteckig ist, **dadurch**, dass die Breite der kürzesten kurzen Seite (11B) des sich verjüngenden Befestigungselements (11) gleich oder größer ist als die Breite des länglichen Befestigungselements und **dadurch**, dass die Länge des länglichen Befestigungselements (10) und die Länge der kürzesten langen Seite (11C) des sich verjüngenden Befestigungselements zwischen 70-100% der Länge der Seitenkanten des Vorderabschnitts (4) liegt.
